# EUROPEAN PATENT APPLICATION

(11) **EP 4 512 325 A1**
(43) Date of publication of application: **26.02.2025**
(21) Application number: 24195633.3
(22) Date of filing: 21.08.2024
(51) Int. Cl.: A61B 5/145, A61B 5/1495, A61B 5/00

(54) **CONTINUOUS GLUCOSE MONITOR**

(30) Priority: 23.08.2023 CN 202311068111
(71) Applicant: Shenzhen Goodix Technology Co., Ltd., Shenzhen, Guangdong 518045 (CN)
(72) Inventor: CHENG, Shuqing, Shenzhen, 518045 (CN); ZENG, Weiping, Shenzhen, 518045 (CN)
(74) Representative: Kraus & Lederer PartGmbB

(57) **Abstract**

A continuous glucose monitor, comprising a housing and a circuit board arranged in the housing, wherein an electronic component is carried on the circuit board; a probe assembly configured to penetrate skin and connected to the circuit board; and an auxiliary sensor connected to the circuit board and configured to detect a compressed state of the continuous glucose monitor. The continuous glucose monitor provided in the present disclosure can identify its own compressed state, thereby improving the accuracy of glucose measurement.

## Description

### TECHNICAL FIELD

The present disclosure relates to the technical field of glucose monitoring, and more particularly relates to a continuous glucose monitor.

### BACKGROUND

A continuous glucose monitor (CGM) is a glucose measuring device that continuously measures glucose concentration in blood through a subcutaneously implanted sensor, and can provide a user with continuous and dynamic glucose data. Compared with conventional glucose monitoring, which requires sampling fingertip blood for measurement, the CGM not only can greatly improve life experience of diabetics, but also can provide more complete and more dynamic glucose data, thus providing more basis for medication and treatment of the diabetics. The CGM is generally implanted on an outer side of an arm. When a user is sleeping, the CGM will be compressed in some sleeping postures (such as side lying), thereby resulting in poor circulation of blood and tissue fluid in the arm, and sharply increasing glucose measurement errors.

Therefore, how to identify a compressed state of the CGM to improve accuracy of glucose measurement is a technical problem to be urgently solved.

### SUMMARY

A continuous glucose monitor provided in a first aspect of embodiments of the present disclosure comprises:
a housing and a circuit board arranged in the housing, wherein an electronic component is carried on the circuit board;
a probe assembly configured to penetrate skin and connected to the circuit board; and
an auxiliary sensor connected to the circuit board and configured to detect a compressed state of the continuous glucose monitor.

In a possible embodiment, the auxiliary sensor is a capacitive sensor, and when the capacitive sensor is subjected to compression, a capacitance value detected by the capacitive sensor will change.

In a possible embodiment, the auxiliary sensor is a pressure sensor, and when the pressure sensor is subjected to compression, a pressure value detected by the pressure sensor will change.

In a possible embodiment, the capacitive sensor is arranged in the housing, and is electrically connected to the circuit board.

In a possible embodiment, the probe assembly comprises a probe that extends to outside through an opening at one end of the housing.

In a possible embodiment, the capacitive sensor and the probe assembly are arranged on a same surface of the circuit board.

In a possible embodiment, the capacitive sensor and the probe assembly are arranged on different surfaces of the circuit board.

In a possible embodiment, the capacitive sensor is arranged on a side edge of the circuit board, and the probe assembly is arranged on a lower surface of the circuit board.

In a possible embodiment, the circuit board is a printed circuit board.

In a possible embodiment, the electronic component comprises a microcontroller unit.

The embodiments of the present disclosure provide a continuous glucose monitor, which can identify its own compressed state, thereby improving the accuracy of blood glucose measurement.

### DESCRIPTION OF DRAWINGS

FIG. 1 is a schematic structural diagram of a continuous glucose monitor provided in an embodiment of the present disclosure.
FIG. 2 is a schematic diagram of a body posture of a user.
FIG. 3 is a schematic diagram of another body posture of a user.
FIG. 4 is a capacitance value variation diagram of a continuous glucose monitor when being non-compressed and when being compressed provided in an embodiment of the present disclosure.

### DETAILED DESCRIPTION

Technical solutions of embodiments of the present disclosure will be clearly and completely described below with reference to the drawings.

The terms used in the present disclosure are intended merely to describe particular embodiments, and are not intended to limit the present disclosure. The singular forms of "a" and "the" used in the present disclosure and the appended claims are also intended to include plural forms, unless the context clearly indicates other meanings.

In addition, the terms such as "first" and "second" are only used for distinguishing between similar objects, and cannot be understood as indicating or implying relative importance or implicitly indicating the number of indicated technical features. Thus, features defined with "first," "second," or the like may explicitly or implicitly include one or more of the features.

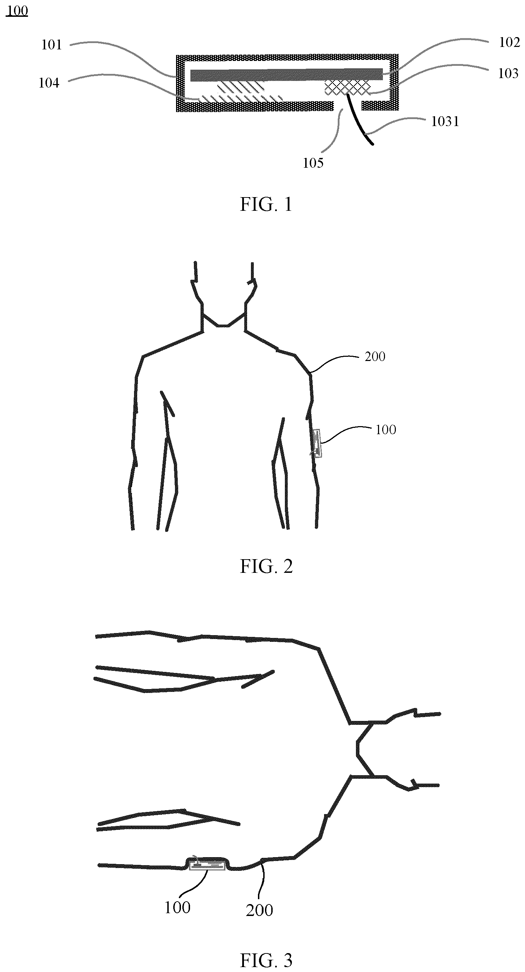

FIG. 1 shows a schematic structural diagram of a continuous glucose monitor provided in an embodiment of the present disclosure.

As shown in FIG. 1, the continuous glucose monitor 100 comprises a housing 101 and a circuit board 102 arranged in the housing, wherein an electronic component is provided in the circuit board 102;

a probe assembly 103 configured to penetrate skin, the probe assembly 103 being connected to the circuit board 102; and

an auxiliary sensor 104 connected to the circuit board 102, the auxiliary sensor 104 being configured to detect a compressed state of the continuous glucose monitor 100.

The continuous glucose monitor 100 in an embodiment of the present disclosure can identify its own compressed state, thereby improving the accuracy of glucose measurement.

Optionally, the circuit board 102 may be a printed circuit board (PCB). The printed circuit board not only can be electrically connected to the probe assembly 103 and the auxiliary sensor 104, but also can play a certain supporting role for the probe assembly 103 and the auxiliary sensor 104.

As an optional embodiment, the electronic component carried on the circuit board 102 includes a microcontroller unit (MCU). After detecting that the continuous glucose monitor 100 is in the compressed state, the auxiliary sensor 104 can transmit compressed state information to the microcontroller unit, and the microcontroller unit can compensate for and correct a blood glucose measurement result based on the received compressed state information to avoid occurrence of a very large measurement error, thereby improving the accuracy of glucose measurement.

As another optional embodiment, after detecting that the continuous glucose monitor 100 is in the compressed state, the auxiliary sensor 104 can transmit the compressed state information to cloud software, and the cloud software can compensate for and correct a blood glucose measurement result based on the received compressed state information to avoid occurrence of a very large measurement error, thereby improving the accuracy of glucose measurement.

As an optional embodiment, the auxiliary sensor 104 is a capacitive sensor, and when the capacitive sensor is subjected to compression, a capacitance value detected by the capacitive sensor will change. By detecting the capacitance value of the capacitive sensor, whether the continuous glucose monitor 100 is subjected to compression can be determined, and the information can be transmitted to the MCU or the cloud software to correct the glucose measurement result.

Optionally, the capacitive sensor is arranged in the housing 101, and is electrically connected to the circuit board 102. The housing 101 can provide certain support and protection for the capacitive sensor arranged therein. Preferably, the capacitive sensor is arranged inside the housing 101 on one side close to skin during use by a user, so that the capacitive sensor can achieve better capacitance detection effects in most usage scenarios.

Optionally, the electronic component carried on the circuit board 102 further comprises a capacitance detection chip, and the capacitive sensor can be connected to the capacitance detection chip on the circuit board 102 through leads.

Optionally, referring to FIG. 1, the probe assembly 103 comprises a probe 1031, the probe 1031 extends to the outside through an opening 105 at one end of the housing 101, so that the probe 1031 can be very easily implanted under the skin.

As an optional embodiment, the capacitive sensor and the probe assembly 103 are arranged on a same surface of the circuit board 102. When the user uses the continuous glucose monitor 100, the side where the probe assembly 103 is located is the side close to the user's skin, and the capacitive sensor and the probe assembly 103 are arranged on the same surface of the circuit board 102, so that the capacitive sensor is also arranged on the side close to the user's skin, thereby achieving better capacitance detection effects in most usage scenarios during use, e.g., on an arm or an abdominal surface.

As another optional embodiment, the capacitive sensor and the probe assembly 103 are arranged on different surfaces of the circuit board 102. In this embodiment, when the user uses the continuous glucose monitor 100, the side where the probe assembly 103 is located is still the side close to the user's skin, but the capacitive sensor is arranged on one side away from the user's skin. Such an arrangement can achieve better capacitance detection effects in some particular usage scenarios during use, e.g., at an underarm site or an intercrural site.

As another optional embodiment, the capacitive sensor is arranged on a side edge of the circuit board 102, and the probe assembly 103 is arranged on a lower surface of the circuit board 102. Based on different product designs, the capacitive sensor can be arranged on the side edge of the circuit board 102, thereby reducing the thickness of the continuous glucose monitor 100, and improving the user experience.

As an optional embodiment, the auxiliary sensor 104 is a pressure sensor, and when the pressure sensor is subjected to compression, a pressure value detected by the pressure sensor will change. By detecting the pressure value of the pressure sensor, whether the continuous glucose monitor 100 is subjected to compression can be determined, and the information can be transmitted to the MCU or the cloud software to correct the glucose measurement result.

It is worth noting that the relevant settings for the capacitive sensor in the embodiments of the present disclosure can also be used for the pressure sensor.

FIG. 2 shows a schematic diagram of a body posture of a user.

As shown in FIG. 2, after the user normally wears the continuous glucose monitor 100 on his arm, if the user is in a body posture, such as standing, sitting, or flat lying, the continuous glucose monitor 100 will not be compressed. When the auxiliary sensor 104 is a capacitive sensor, in this case, a signal sensed by the capacitive sensor is a capacitance value formed between the capacitive sensor and user's skin 200.

FIG. 3 shows a schematic diagram of another body posture of a user.

As shown in FIG. 3, after the user normally wears the continuous glucose monitor 100 on his arm, if the user is in a body posture of side lying, the continuous glucose monitor 100 will sink into the user's skin 200 because the skin on the arm is soft, thereby producing the effect of being wrapped. When the auxiliary sensor 104 is a capacitive sensor, in this case, the capacitance value formed between the capacitive sensor and the user's skin 200 will be significantly increased. This feature can be used to implement compression detection of the continuous glucose monitor 100.

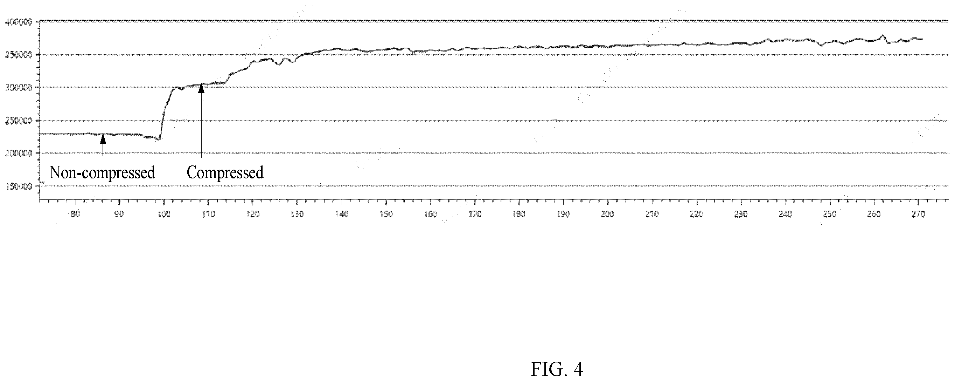

FIG. 4 shows a capacitance value variation diagram of a continuous glucose monitor when being non-compressed and when being compressed provided in an embodiment of the present disclosure. The auxiliary sensor 104 inside the continuous glucose monitor 100 is a capacitive sensor.

As shown in FIG. 4, abscissas in the figure represent sampling points (33 sampling points per second), and ordinates represent capacitance values. Before the 100th sampling point, the continuous glucose monitor 100 is not compressed, and the capacitance value of the capacitive sensor is in a stable state. After the 100th sampling point, the continuous glucose monitor 100 is subjected to compression, and the capacitance value of the capacitive sensor is significantly increased. Therefore, the capacitance value changes of the capacitive sensor can be detected to identify the compressed state of the continuous glucose monitor 100.

In a preferred embodiment of the present disclosure, a capacitive sensor is additionally provided in the continuous glucose monitor 100, to identify the compressed state of the continuous glucose monitor 100 using the characteristic that the capacitance of the capacitive sensor is increased in the compressed state, and then provide the compressed state information to a MCU or a host computer, so that the MCU or the host computer can compensate for and correct the glucose measurement result based on the compressed state information, thereby improving the accuracy of glucose measurement.

The preferred embodiments of the present disclosure are described in detail above with reference to the drawings. However, the present disclosure is not limited to the specific details of the above-mentioned embodiments. Within the scope of the technical concept of the present disclosure, multiple simple modifications can be made to the technical solutions of the present disclosure, and the simple modifications are all encompassed within the scope of protection of the present disclosure.

## Claims

1. A continuous glucose monitor, comprising:
a housing and a circuit board arranged in the housing, wherein an electronic component is carried on the circuit board;
a probe assembly configured to penetrate skin and connected to the circuit board; and
an auxiliary sensor connected to the circuit board and configured to detect a compressed state of the continuous glucose monitor.

2. The continuous glucose monitor according to claim 1, wherein the auxiliary sensor is a capacitive sensor, and when the capacitive sensor is subjected to compression, a capacitance value detected by the capacitive sensor changes.

3. The continuous glucose monitor according to claim 1, wherein the auxiliary sensor is a pressure sensor, and when the pressure sensor is subjected to compression, a pressure value detected by the pressure sensor changes.

4. The continuous glucose monitor according to claim 2, wherein the capacitive sensor is arranged in the housing, and is electrically connected to the circuit board.

5. The continuous glucose monitor according to claim 4, wherein the probe assembly comprises a probe that extends to outside through an opening at one end of the housing.

6. The continuous glucose monitor according to claim 5, wherein the capacitive sensor and the probe assembly are arranged on a same surface of the circuit board.

7. The continuous glucose monitor according to claim 5, wherein the capacitive sensor and the probe assembly are arranged on different surfaces of the circuit board.

8. The continuous glucose monitor according to claim 5, wherein the capacitive sensor is arranged on a side edge of the circuit board, and the probe assembly is arranged on a lower surface of the circuit board.

9. The continuous glucose monitor according to any one of claims 1 to 8, wherein the circuit board is a printed circuit board.

10. The continuous glucose monitor according to any one of claims 1 to 8, wherein the electronic component comprises a microcontroller unit.
